# EUROPEAN PATENT SPECIFICATION

(11) **EP 2 315 146 B1**
(45) Date of publication and mention of the grant of the patent: **12.08.2015**
(21) Application number: 09171951.8
(22) Date of filing: 01.10.2009
(51) Int. Cl.: G06F 19/00

(54) **Method and apparatus for monitoring and controlling a medical device using a wireless mobile communication device**
Verfahren und Vorrichtung zur Überwachung und Steuerung einer medizinischen Vorrichtung mithilfe einer drahtlosen mobilen Kommunikationsvorrichtung
Procédé et appareil de surveillance et de contrôle d'un dispositif médical utilisant un dispositif de communication mobile sans fil

(43) Date of publication of application: 27.04.2011
(73) Proprietor: BlackBerry Limited, Waterloo, ON N2K 0A7 (CA)
(72) Inventor: Waniss Amgad Mofied, Waterloo Ontario N2L 5Z5 (CA)
(74) Representative: MERH-IP Matias Erny Reichl Hoffmann

(56) References cited:
- WO-A1-2008/097316
- WO-A1-2009/050477
- WO-A1-2009/063303
- WO-A2-2004/070995
- WO-A2-2009/071205

## Description

### Technical Field

The present disclosure relates generally to medical devices, and more particularly to a method and apparatus for controlling a medical device using a wireless mobile communication device.

### Background

Medical devices such as insulin pumps and heart-regulation devices are commonly used by patients who may be unable to independently monitor, react to and/or operate such devices (e.g. patients who are infirm, elderly or children). In situations such as this, the patients must depend on caregivers to oversee their treatment using such devices. For example, a young child who has been diagnosed with "type I" diabetes may receive treatment using an insulin pump for real-time supply of insulin to regulate the child's blood glucose levels. The child's parents and/or caregivers monitor glucose levels, make decisions on how much insulin is required, and then control the pump to deliver the required flow of insulin. It will be appreciated that the patient is therefore highly dependent on caregivers to provide life-critical care.

In an effort to provide some measure of flexibility and mobility to caregivers, systems are known for providing remote monitoring of a patient by a caregiver. For example, United States Patent Publication No. 2008/0119705 discloses a connector that may be attached to a consumer electronic device, such as a cellular telephone, personal digital assistant, etc., to allow communication between the consumer electronic device and a medical device, such as an infusion device, implantable pump, glucose meter, etc. WO 2009/063303 A1 discloses systems and methods of providing telemedicine services comprising a medical device that obtains diagnostic information a gateway device coupled to the medical device, an application server coupled to the gateway device via wireless and wired networks, a database coupled to the application server, the database storing the diagnostic information, and an analyzing device coupled to the database, the analyzing device analyzes records in the database to identify diagnostic information that exceeds predefined thresholds.

WO 2008/097316 discloses a method of operating a medical device connector as in the characterising part of claim 1 and a method of operating a wireless mobile communication device as in the characterising part of claim 6, and the respective devices as in the characterising part of claims 3 and 8.

### Background

According to an aspect of this specification, there is provided a method for monitoring and controlling a medical device using at least one of a plurality of wireless mobile communication devices, comprising transmitting an add request to one of said plurality of wireless mobile communication devices, said add request including an ID and a request key; receiving a device-ID and authentication key from said one of said plurality of wireless mobile communication devices; processing said authentication key to determine if said wireless mobile communication device is an authenticated wireless mobile communication device and if said wireless mobile communication device is authenticated then assigning a role to said wireless mobile communication device, storing said device-ID, authentication key and role in a list of authenticated wireless mobile communication devices, and transmitting a confirmation to said wireless communication device, wherein said role includes permissions for monitoring and controlling said medical device, and if said wireless mobile communication device is not authenticated then transmitting a request failure to said wireless mobile communication device; and enabling at least one of monitoring data from said medical device and controlling said medical device by said authenticated wireless mobile communication device.

According to another aspect, there is provided a medical device connector for facilitating monitoring and control of a medical device by at least one of a plurality of wireless mobile communication devices, comprising a memory; a communication subsystem for transmitting an add request to one of said plurality of wireless mobile communication devices, said add request including an ID and a request key, and receiving a device-ID and authentication key from said one of said plurality of wireless mobile communication devices; a microprocessor executing a software application for processing said authentication key to determine if said wireless mobile communication device is an authenticated wireless mobile communication device and if said wireless mobile communication device is authenticated then assigning a role to said wireless mobile communication device, storing said device-ID and authentication key in a list of authenticated wireless mobile communication devices within said memory, and transmitting a confirmation to said wireless communication device, said role including permissions for monitoring and controlling said medical device, and if said wireless mobile communication device is not authenticated then transmitting a request failure to said wireless mobile communication device via said communication subsystem; and a data port for monitoring data from said medical device and transmitting data received from said authenticated wireless mobile communication device via said communication subsystem for controlling said medical device.

According to a further aspect of this specification, there is provided a method of operating a wireless mobile communication device for monitoring and control of a medical device connected to a medical device connector, comprising receiving an add request from said medical device connector, said add request including an ID and a request key; processing said add request and in response generating a device-ID and authentication key; transmitting said device-ID and authentication key to said medical device connector; receiving one of either a confirmation that said wireless mobile communication device has been authenticated by said medical device connector, said confirmation including a medical connector device-ID and a role assigned to said wireless mobile communication device, or a request failure in the event said wireless mobile communication device is not authenticated by said medical device connector; and in the event of receipt of said confirmation then storing said medical device connector ID assigned role and authentication key in a list of authenticated medical device connectors; or in the event of receipt of said request failure then logging said failure.

According to yet another aspect of this specification, there is provided a mobile communication device for facilitating monitoring and control of a medical device via a medical device connector, comprising a memory; a communication subsystem for receiving an add request from said medical device connector, said add request including an ID and a request key; a microprocessor executing a software application for processing said add request and in response generating a device-ID and authentication key, transmitting said device-ID and authentication key to said medical device connector and receiving via said communication subsystem one of either a confirmation that said wireless mobile communication device has been authenticated by said medical device connector, said confirmation including a medical connector device-ID and a role assigned to said wireless mobile communication device, or a request failure in the event said wireless mobile communication device is not authenticated by said medical device connector, and storing said one of either said confirmation and authentication key or said request failure in said memory.

### Brief Description of the Drawings

FIG. 1 is a block diagram of an exemplary or illustrative system for monitoring and controlling a medical device using wireless mobile communication devices, in accordance with the present disclosure;
FIG. 2 is block diagram illustrating a wireless mobile communication device in accordance with the present disclosure;
FIG. 3 is a block diagram of an exemplary medical device connector to the medical device for facilitating communication with a wireless mobile communication device in accordance with the present disclosure;
FIG. 4 is a flow diagram showing configuration of a wireless mobile communication device to communicate with the medical device, via a medical device connector, in a predetermined role, thereby allowing a caregiver to remotely monitor and control the medical device;
FIG. 5 is a flow diagram showing monitoring, via the medical device connector, of data from a medical device by an authenticated wireless mobile communication device and presentation of the data thereon in accordance with the present disclosure;
FIG. 6 is a flow diagram showing control of the medical device, via a medical device connector, by the authenticated wireless mobile communication device in accordance with the present disclosure; and
FIGS. 7 and 8 are flow diagrams showing two techniques of pushing of data from the medical device to an authenticated wireless mobile communication device, via the medical device connector, and presentation of the data thereon in accordance with the present disclosure.

### Detailed Description

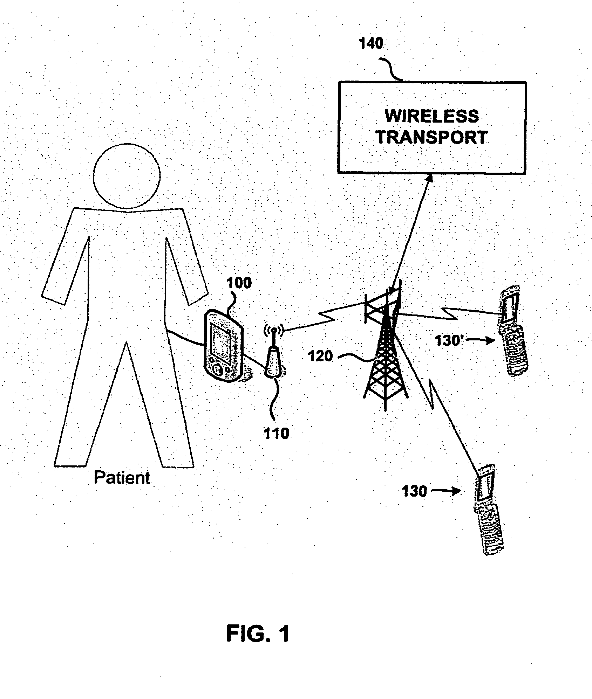

FIG. 1 shows a system for enabling multiple caregivers to communicate with and control a medical device 100 via their wireless mobile communication devices 130, 130', etc. As discussed above, medical device 100 may be any device that provides treatment to a patient, such as an insulin pump, heart-regulation device, etc. According to an exemplary embodiment, an intelligent medical device connector (MDC) 110 is connected to the device 100 in order to facilitate communication over a wireless network 120 with caregiver mobile communication devices 130, 130'. Alternatively, the functionality of MDC 110 may be incorporated into the device 100, and a typical user may perceive medical device 100 and MDC 110 as a single device. For purposes of illustration, medical device 100 and MDC 110 will be described as distinct devices. Wireless communications over network 120 are controlled by a wireless transport 140, in a well-known manner.

The illustrated system obviates the need of the caregiver(s) to be physically present with the patient at all times in order to control the device 100. The caregiver(s) is (are) able to remotely control the device 100 in order to obtain medical data (e.g. glucose levels, blood pressure, pulse, etc.) from the device 100 and to control operation of the device (e.g. insulin output, pacemaker rhythm, etc.) from a remote location. Moreover, as discussed in greater detail below, according to an exemplary embodiment, wireless transport 140 provides secure authentication and selection between multiple caregiver devices 130, 130', etc., so that responsibility for monitoring and treating the patient can be safely and securely shared.

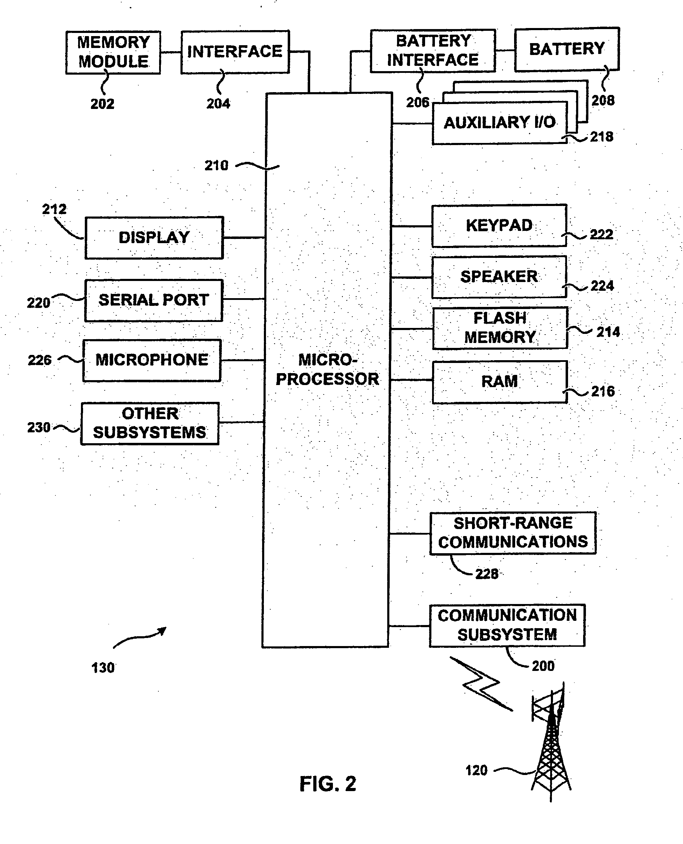

FIG. 2 shows a block diagram illustrating some of the components of an illustrative wireless mobile communication devices 130, 130', etc. In the embodiment depicted in FIG. 2, wireless mobile communication device 130 includes a communication subsystem 200 for wireless two-way data and voice communication with the wireless network 120. Communication subsystem 200 may include one or more receivers, transmitters, antennas, signal processors and other components associated with wireless communications. The particular design of the communication subsystem 200 depends on the network in which the wireless mobile communication device 130 is intended to operate. The concepts herein may be applicable to a variety of wireless mobile communication devices, such as two-way pagers, cellular telephones, etc.

In the embodiment shown in FIG. 2, network access is associated with a subscriber or user of the wireless mobile communication device 130 via a memory module 202, which may be a Subscriber Identity Module (SIM) card for use in a GSM network or a Universal Subscriber Identity Module (USIM) card for use in a Universal Mobile Telecommunication System (UMTS). The SIM card is inserted in or connected to an interface 204 of the wireless mobile communication device 130 to operate in conjunction with the wireless network 120. Alternatively, the wireless mobile communication device 130 may have an integrated identity module for use with systems such as Code Division Multiple Access (CDMA) systems.

The wireless mobile communication device 130 also includes a battery interface 206 for receiving at least one rechargeable battery 208. The battery 208 provides electrical power to at least some of the electrical circuitry in the wireless mobile communication device 130, and the battery interface 206 provides a mechanical and electrical connection for the battery 208.

The wireless mobile communication device 130 includes a microprocessor 210 which controls the overall operation of the device. Communication functions, including at least data and voice communications, and which may include the data communications pertaining to glucose levels as discussed in more detail below, are performed through the communication subsystem 200, as discussed above. The microprocessor 210 also interacts with additional device subsystems such as a display 212, flash memory 214, a random access memory (RAM) 216, auxiliary input/output (I/O) subsystems 218, a data port such as serial port 220, keypad 222, speaker 224, microphone 226, a short-range (i.e. near field) communications subsystem 228, and any other device subsystems generally designated as 230. The microprocessor may further interact with other components, which for simplicity are not shown in FIG. 2.

The microprocessor 210, in addition to its operating system functions, enables execution of software applications on the wireless mobile communication device 130. Software, which may include operating system software or application software, may be stored in flash memory 214, RAM 216 or any other memory element. As will be discussed below, according to an exemplary embodiment, application software is provided to permit the wireless mobile communication device 130 to monitor data received from MDC 110 and provide signals for controlling the medical device 100 via the MDC 110 of FIG. 1. This application software may be stored in any memory element of the wireless mobile communication device 130, or any medium configured to store machine-executable instructions that can be carried out by the microprocessor 210.

A predetermined set of applications that control basic device operations, including data and voice communication applications, will normally be installed on the wireless mobile communication device 130 during or after manufacture. The wireless mobile communication device 130 may include a personal information manager (PIM) application having the ability to organize and manage data items relating to a user such as, but not limited to, instant messaging, email, calendar events, voice mails, appointments, and task items.

For voice communications, the wireless mobile communication device 130 may receive one or more signals associated with a voice communication, such as an indication of the identity of a calling party. In response to the received signals, the microprocessor 210 may generate output for display on display 212 and/or the speaker 224.

In a data communication mode, a received data signal representing information such as a glucose levels, is received and processed by the communication subsystem 200 and input to the microprocessor 210, which further processes the signal. In response to the received data signal, the microprocessor 210 may generate output for display on the display 212 (e.g. a graphical representation of current and historical glucose levels).

In addition, as discussed briefly above, a short-range communications subsystem 228 is provided for communication between the wireless mobile communication device 130 and different systems or devices, which need not necessarily be similar devices. For example, the short-range communications subsystem 228 may include an infrared device and associated circuits and components, or a wireless bus protocol compliant communication mechanism such as a Bluetooth^{™} communication module to provide for communication with similarly-enabled systems and devices. In another embodiment, the short-range communications subsystem 228 may be a wireless networking communications subsystem, conforming to IEEE 802.11 standards such as one or more of 802.11b, 802.11g, or 802.11n. Data communications pertaining to glucose levels may also be sent to or received by the short-range communications subsystem 228, but in typical operation, data communications pertaining to glucose levels may be sent to or received by the communication subsystem 200.

The wireless mobile communication devices 130, 130', etc may include one or more circuit boards (not shown) that implement the components described above. This disclosure is not limited to any particular electronic component or software module or any combination thereof.

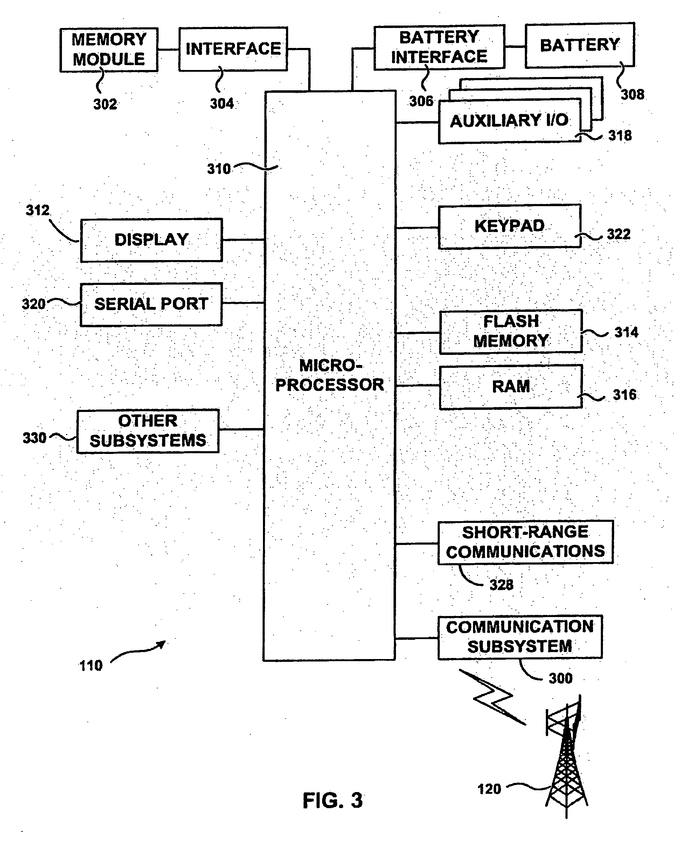

FIG. 3 shows a block diagram illustrating components of the MDC 110 for facilitating communication between medical device 100 and the wireless mobile communication devices 130, 130', etc. In the illustrative embodiment of FIG. 3, the functional components of MDC 110 are similar to those of the mobile communication device 130 of FIG. 2, with the exception that there is no provision for voice communications and the user interface is greatly simplified. Thus, according to the illustrative embodiment of FIG. 3, the MDC 110 is a 'stripped down' version of mobile communication device 130. The concepts described herein are not limited, however, to an MDC that is a 'stripped down' version of mobile communication device 130.

In the embodiment depicted in FIG. 3, MDC 110 includes a communication subsystem 300 for wireless two-way data and voice communication with the wireless network 120. Communication subsystem 300 may include one or more receivers, transmitters, antennas, signal processors and other components associated with wireless communications. The particular design of the communication subsystem 300 depends on the network in which the MDC 110 is intended to operate (e.g. wireless cellular network).

In the embodiment shown in FIG. 3, network access is associated with a subscriber or user of the MDC 110 via a memory module 302, which may be a Subscriber Identity Module (SIM) card for use in a GSM network or a Universal Subscriber Identity Module (USIM) card for use in a Universal Mobile Telecommunication System (UMTS). The SIM card is inserted in or connected to an interface 304 of the MDC 110 to operate in conjunction with the wireless network 120. Alternatively, the MDC 110 may have an integrated identity module for use with systems such as Code Division Multiple Access (CDMA) systems.

The MDC 110 also includes a battery interface 306 for receiving at least one rechargeable battery 308. The battery 308 provides electrical power to at least some of the electrical circuitry in the MDC 110, and the battery interface 306 provides a mechanical and electrical connection for the battery 308.

The MDC 110 includes a microprocessor 310 which controls the overall operation of the device. Communication functions, including at least data communications, are performed through the communication subsystem 300, as discussed above. The microprocessor 310 also interacts with additional device subsystems such as a display 312, flash memory 314, a random access memory (RAM) 316, auxiliary input/output (I/O) subsystems 318, a data port such as serial port 320 for connection to the medical device 100, optional keypad 322, a short-range (i.e. near field) communications subsystem 328 which can serve as an alternative data port to the serial port 320, for wireless communication with medical device 100 (e.g. via Bluetooth®, WiFi, etc.), and any other device subsystems generally designated as 330. The microprocessor 310 may further interact with other components, which for simplicity are not shown in FIG. 3.

The microprocessor 310, in addition to its operating system functions, enables execution of software applications on the MDC 110. Software, which may include operating system software or application software, may be stored in flash memory 314, RAM 316 or any other memory element. As will be discussed below, according to an exemplary embodiment, application software is provided to permit the MDC 110 to receive data from and provide control signals for control the medical device 100 of FIG. 1. A predetermined set of applications that control basic device operations, including data communication applications, will normally be installed on the MDC 110 during or after manufacture.

In operation, according to an exemplary embodiment, data/command signals are exchanged between the medical device 100 and MDC 110 via a wired or wireless connection, such as serial port 320 for receiving information such as a glucose levels that is then wirelessly transmitted to a mobile communication device 130, 130', etc. via the communication subsystem 300, and for controlling the device 100 (e.g. administer a regulated dosage) under control of mobile communication device 130, 130', etc.

In another embodiment, data/command signals may be exchanged via short-range communications subsystem 328, for example using an infrared device and associated circuits and components, or a wireless bus protocol compliant communication mechanism such as a Bluetooth^{™} communication module to provide for communication with similarly-enabled systems and devices. In a further embodiment, the short-range communications subsystem 328 may be a wireless networking communications subsystem, conforming to IEEE 802.11 standards such as one or more of 802.11b, 802.11g, or 802.11n. In yet another embodiment, data/command signals are exchanged via any other suitable generic data exchange mechanism (e.g. mini-USB, etc.).

The MDC 110 may include one or more circuit boards (not shown) that implement the components described above. This disclosure is not limited to any particular electronic component or software module or any combination thereof.

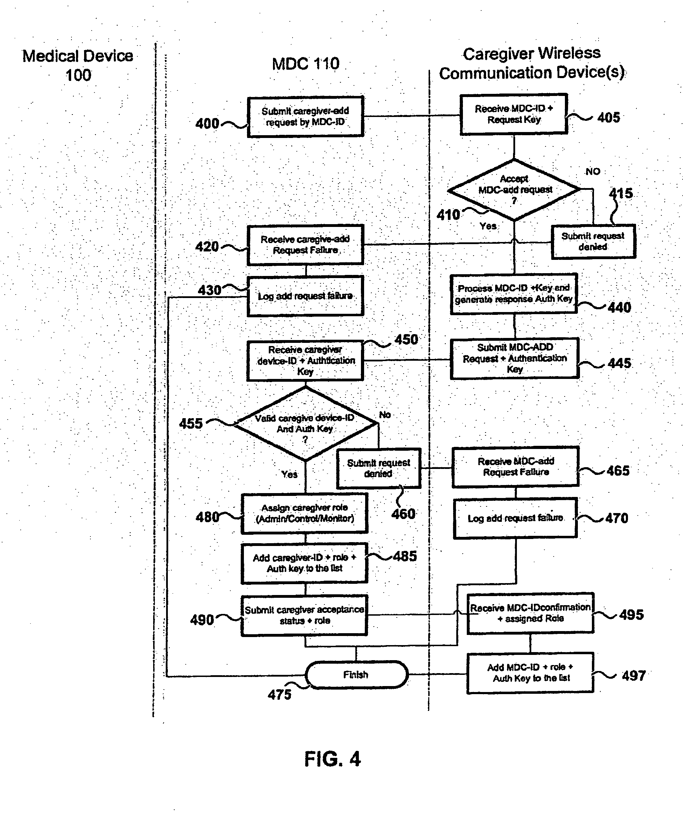

In operation, with reference to the flowchart of FIG. 4, a plurality of wireless mobile communication devices 130, 130', etc., of FIGS. 2 and 3 may be configured permitting multiple caregivers to remotely monitor and control a medical device 100 via MDC 110. Specifically, MDC 110 may be caused to submit a request to add a specific caregiver, for example by entering a suitable command via keypad 322. The request to add a specific caregiver may identify a mobile communication device 130 and may also request one or more possible caregiver roles with associated assigned permission, as discussed below. The MDC 110 sends a request message (step 400) to the caregiver's mobile communication device 130. The request message includes an identifier (MDC-ID) for the requesting device 110. The request message is transmitted over the network 120 and received at mobile communication device 130 (step 405). An application executed by microprocessor 210 within device 130 determines whether or not to accept the caregiver request (step 410). If not, wireless mobile communication device 130 transmits a denial message to the MDC 110 (step 415). The denial message is received by the MDC 110 (step 420), which then logs the request failure (step 430) and the communication exchange finishes (step 475). Authentication serves to protect the patient from malevolent acquisition of private medical data and/or potentially life-threatening remote control of the medical device 100.

If the caregiver-add request message is accepted (a "YES" at step 410), the mobile communication device 130 processes the request and generates an authentication key (step 440). The mobile communication device 130 then generates and transmits a MDC-ADD request that contains the caregiver device-ID along with the authentication key (step 445). Information in addition to the MDC-ADD request with the caregiver device-ID and the authentication key may also be transmitted. In the event the mobile communication device 130 is presented with options pertaining to a caregiver role with associated assigned permission, for example, a communication pertaining to a selected caregiver role may be transmitted as well. The MDC-ADD request with caregiver device-ID and authentication key are received by the MDC 110 (step 450), and an application executed by microprocessor 310 within MDC 110 determines whether or not it has received a valid caregiver device-ID and authentication key (step 455). If not (step 460), MDC 110 transmits a failure message which is received by the mobile communication device 130 (step 465). The add request failure message is logged (step 470) and the communication exchange finishes (step 475). Failure to receive a valid caregiver device-ID and authentication key does not lead the MDC 110 to accept commands pertaining to caregiving from the mobile communication device 130.

On the other hand, if a valid caregiver device-ID and authentication key are received (step 480), MDC 110 assigns a requested caregiver role (step 480), such as Admin, Control, Monitor, and adds the caregiver device-ID, role and authentication key to a list (step 485) maintained in memory (e.g., flash memory 314). The assigned role permission data that defines what permissions will be assigned to mobile communication device 130, including permission to monitor status/data of the medical device 100 (i.e. read permission), to control the medical device 100 (i.e. read/write permission), and/or to act as an Administrator (i.e. capable of assigning roles to other authenticated ones of the mobile communication devices 130', etc. The MDC 110 then transmits a message for accepting the caregiver in the requested role (step 490). The caregiver acceptance message (including MDC-ID confirmation and the assigned role) is received by mobile communication device 130 (step 495), and the MDC-ID, role and authentication key are added to a list maintained in memory (e.g. flash memory 214). The communication exchange then finishes (step 475).

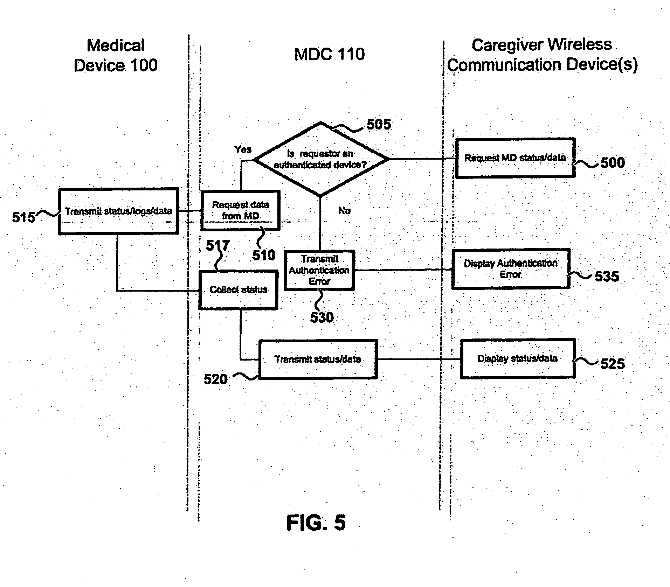

FIG. 5 is a flow diagram showing monitoring of medical device 100 by an authenticated wireless Mobile communication device 130 and presentation of the data thereon. The device 130 sends a request for status/data relating to medical device 100 (step 500), wherein the request includes the device-ID and authentication key. The request is received by MDC 110 which then determines (step 505) if the requesting device 130 is an authenticated device by checking the device-ID and authentication key against the list saved in memory (see step 485). If the device 130 is authenticated (a "YES" at step 505), MDC 110 then requests the relevant data (e.g. blood glucose level, blood pressure, etc.) from medical device 100 (step 510). The device 100 then transmits the requested data to the MDC 110 via serial port 320 or other port (step 515). The MDC 110 wirelessly collects the status data (step 517) and transmits the requested data to mobile communication device 130 (step 520) which in response displays the data for viewing by the caregiver (step 525).

On the other hand, if the caregiver device is not authenticated (i.e. a "NO" at step 505) the MDC 110 transmits an authentication error to the mobile communication device 130 (step 530) which in response generates an error message (step 535). In this event, status data is not collected and transmitted to mobile communication device 130.

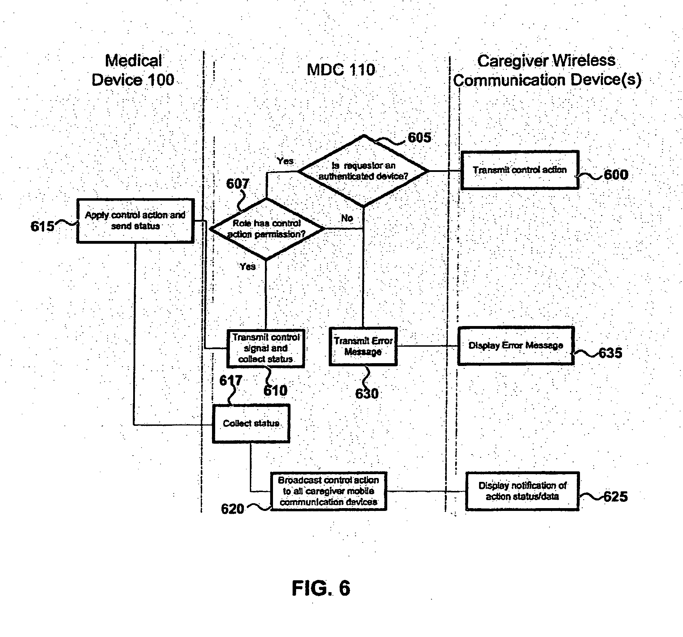

FIG. 6 is a flow diagram showing control of the medical device 100 by an authenticated wireless mobile communication device 130. The device 130 sends a request for control action (step 600) for controlling operation of medical device 100 (e.g. administering a dose of insulin or other i-nedicaLion), wherein the request for control action includes the device-ID and authentication key. The request for control action is received by MDC 110 which then determines (step 605) if the requesting device 130 is an authenticated device by checking the list saved in memory (see step 485). If the device 130 is authenticated (a "YES" at step 605), MDC 110 then determines (step 607) if the role of the authenticated caregiver includes permission to take control action at the medical device 100 (e.g. to administer medication, etc.). If yes, MDC 110 transmits the control action to the medical device 100 (step 610). The device 100 then executes the control action, and transmits status data to the MDC 110 via serial port 320 (step 615), or other suitable communication mechanism. The MDC 110 collects the status data (step 617) and wirelessly broadcasts the resulting status data to all mobile communication devices 130. 130' (step 620) each of which in response displays the data for viewing by the associated caregiver (step 625).

On the other hand, if the caregiver device is not authenticated (i.e. a "NO" at step 605) or does not have permission to control the medical device 100 (i.e. a "NO" at step 607), the MDC 110 transmits an error message to the mobile communication device 130 (step 630) which in response displays the error message (step 635). In this event, the MDC 110 transmits no control action to the medical device 100.

According to one embodiment, redundant, conflicting or overlapping actions transmitted by multiple care giver mobile communication devices 130, 130', etc. to same patient MDC 110 may be resolved by programming into the MDC 110 predetermined governance criteria for handling such multi-sourced requested actions during race conditions, before applying the control action (step 615). Non-exhaustive examples of such governance criteria include predetermined thresholds for accumulative dose intake that can not be exceeded within a certain time period, requesting acknowledgement of a previous pending action transmitted by another care giver, etc.

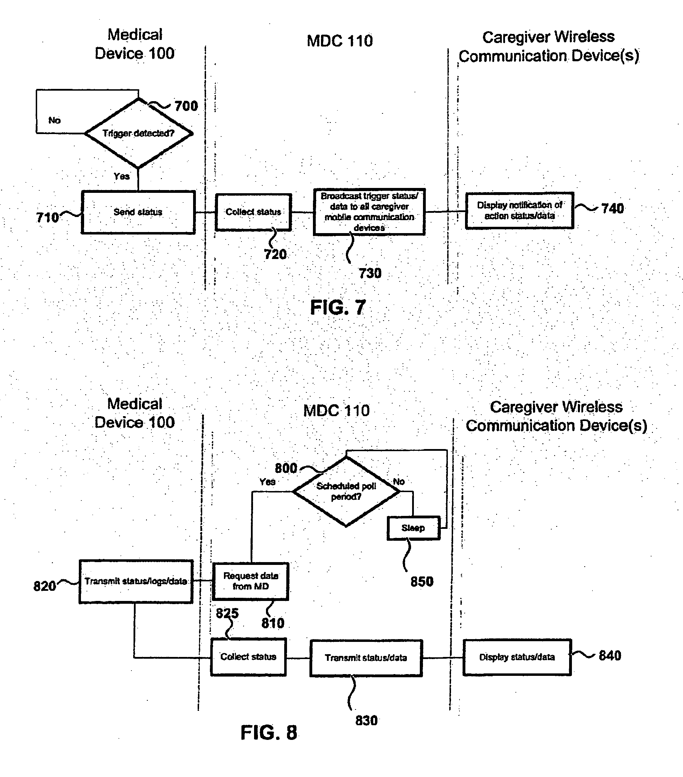

With reference to the flowchart of FIG. 7, MDC 110 may be configured to push data from the medical device 100 to an authenticated wireless mobile communication device 130 in response to a trigger, such as an alarm occurring at the device 100 (e.g. excessively high/low blood glucose levels, excessively high/low blood pressure, excessively high/low pulse, etc.). The device 100 detects the trigger (a "YES" at step 700) and in response transmits a status message to the MDC 110 (step 710) via the serial port 320, or other suitable data exchange mechanism. The MDC 110 collects the status data (step 720) and wirelessly broadcasts the status data to all mobile communication devices 130, 130' (step 730) each of which in response displays the data for viewing by the associated caregiver (step 740) so that at least one of the caregivers can then take remedial action. The mobile communication devices 130, 130' may provide for techniques intended to attract a caregiver's attention to the data, such as an audible alarm, verbal alert, flashing display, device vibration, or the like.

With reference to the flowchart of FIG. 8, MDC 110 may be configured to push data from the medical device 100 to an authenticated wireless mobile communication device 130 as a result of periodic polling of the device 100 by the MDC 110. One the periodic poll period occurs (a "YES" at step 800), MDC 110 requests the relevant data (e.g. blood glucose level, blood pressure, etc.) from medical device 100 (step 810). The device 100 then transmits the requested data to the MDC 110 via serial port 320 (step 820). The MDC 110 tirelessly collects the status data (step 825) and transmits the requested data to mobile communication device 130 (step 830) which in response displays the data for viewing by the caregiver (step 840). The mobile communication devices 130, 130' may provide for techniques for attracting a caregiver's attention to the data.

If the predefined polling period has not yet occurred (a "NO" at step 800), the MDC 110 operates in a low-power sleep mode (step 850), and no status data is collected or transmitted.

The illustrative embodiments set forth above may provide one or more advantages. For example, some embodiments may provide flexibility, in that a variety of wireless mobile communication devices may be employed for monitoring or caregiving via the MDC 110. Flexibility may also be bolstered by the mobility of the mobile communication devices, which need not be in proximity to the. MDC 110 in order to receive data or transmit control actions. Various embodiments also support flexibility in relation to permissions and degrees of caregiving. In some implementations, one mobile communication device may be given one level of permission, and another mobile communication device may be given a different level of permission for the same MDC 110. Further, various embodiments support enhanced security to prevent accidental or otherwise inappropriate remote access to the MDC 110. One or a defined number of authorized caregivers may be granted access. As a consequence, one or more embodiments provide widely scalable applications from individual consumers to Enterprise-like or commercial solutions such as nursing homes and hospitals where both patients and caregivers form part of a single large community of interest. By providing bi-directional closed-loop, secure and well-controlled communications between the MDC 110 and wireless communication devices 130, 130', etc. caregivers in active roles may effectively help infirm patients without necessarily interfering with patient privacy and independence for unrelated life functions. According to some embodiments, multiple care givers can assign and/or exchange roles, provided they are granted appropriate permissions (e.g. supervisor/adtyiinistrator role), remotely from their mobile communication devices 130, 130', etc., so that they collaboratively share the care giving responsibilities to a patient without the patient having to worry about such assignment or coordination. Changes in the roles and permissions can, for example, be logged centrally at the MDC 110 so that traceability and security are maintained.

The embodiments set forth above are for illustration, and although one or more particular embodiments of the system and method have been described herein, changes and modifications may be made thereto. For example, a person of skill in the art will appreciate that the method of configuring a wireless mobile communication device to communicate with the medical device in a predetermined role, as set forth herein, may be extended to the control of multiple patients by a single caregiver via a wireless mobile communication device, or the control of multiple patients by multiple caregivers via respective wireless mobile communication devices, using the mapping process of FIG. 4 with appropriate request/authentication/role-granting steps for multiple patient medical devices and caregiver(s) communication device(s).

Also, although the exemplary embodiment has been described in terms of providing care to a diabetic child patient receiving treatment via an insulin pump, the principles set forth herein may be applied to other medical scenarios where remote care giving would be advantageous (e.g. patients using ventilators, implanted cardiac defibrillators, dialysis devices, etc.). All such embodiments and applications are believed to be within the scope of this disclosure in its broadest aspects and as set forth in the following claims.

## Claims

1. A method of operating a medical device connector (110) to monitor and control at least one medical device (100) using at least one of a plurality of wireless mobile communication devices (130), comprising:
transmitting (400) an add request to one of said plurality of wireless mobile communication devices (130), said add request including an ID and a request key;
receiving (450) a device-ID and authentication key from said one of said plurality of wireless mobile communication devices (130);
processing (455) said authentication key to determine if said wireless mobile communication device is an authenticated wireless mobile communication device (130) and if said wireless mobile communication device is not authenticated then transmitting a request failure to said wireless mobile communication device;
enabling at least one of monitoring data (510) from said at least one medical device (100) and controlling (610) said at least one medical device by said authenticated wireless mobile communication device (130);
wherein said monitoring data (510) from said at least one medical device (100) further comprises:
receiving a request (500) from said wireless mobile communication device (130) for data relating to said at least one medical device (100), said request including said device-ID and authentication key;
in response to said received request for data relating to said medical device (100), checking (505) a list of authenticated wireless mobile communication devices to determine if said device-ID and authentication key indicate that said wireless mobile communication device (130) is one of said authenticated wireless mobile communication devices; and
if said the wireless mobile communication device (130) is one of said authenticated wireless mobile communication devices then collecting (517) said data from said at least one medical device and wirelessly transmitting (520) said data to said wireless mobile communication device (130), and otherwise transmitting an authentication error (530) to said wireless mobile communication device; and
wherein said controlling (610) said at least one medical device by said authenticated wireless mobile communication device (130) further comprises:
receiving a control action (600) from said wireless mobile communication device (130) for controlling said at least one medical device (100), said control action including device-ID and authentication key; and
In response to said received control action, checking (605, 607) said list to determine if said device-ID and authentication key indicate that said wireless mobile communication device (130) is one of said authenticated wireless mobile communication devices;
**characterized in that**
said add request includes a role,
said role includes permissions for monitoring and controlling said at least one medical device,
said role includes a permission to take said control action,
said processing (455) said authentication key to determine if said wireless mobile communication device is an authenticated wireless mobile communication device comprises:
if said wireless mobile communication device is authenticated then assigning (480) the role to said wireless mobile communication device, storing (485) said device-ID, authentication key and role in said list, and transmitting (490) a confirmation to said wireless mobile communication device,
said controlling (610) said at least one medical device by said authenticated wireless mobile communication device (130) further comprises:
if said wireless mobile communication device is one of said authenticated wireless mobile communication devices (130) and said role includes permission to take said control action then transmitting said control action (610) to said at least one medical device (100), collecting data (617) from said at least one medical device following said control action, and wirelessly transmitting said data (620) to said wireless mobile communication device; and otherwise transmitting an error message (630) to said wireless mobile communication device.

2. The method of claim 1 wherein said at least one medical device (100) is an insulin pump, said monitoring of data includes monitoring blood glucose level and said controlling includes administering insulin via said insulin pump.

3. A medical device connector (110) to monitor and control at least one medical device (100) by at least one of a plurality of wireless mobile communication devices, comprising:
a memory (314);
a communication subsystem (300);
a microprocessor (310) executing a software application for causing the medical device connector (110) to perform the method of claim 1; and
a data port (320).

4. The medical device connector of claim 3 wherein said communication subsystem (300) includes one or more receivers, transmitters, antennas, signal processors and other components associated with wireless communications.

5. The medical device connector of claim 3 wherein said data port (320) is a serial port.

6. A method of operating a wireless mobile communication device (130) to monitor and control at least one medical device (100) connected to a medical device connector (110), comprising:
receiving (405) an add request from said medical device connector (110), said add request Including an ID, and a request key;
processing (440) said add request and in response generating a device-ID and authentication key;
transmitting (445) said device-ID and authentication key to said medical device connector (110);
receiving one of either a confirmation (495) that said wireless mobile communication device (130) has been authenticated by said medical device connector (110), or a request failure (465) in the event said wireless mobile communication device is not authenticated by said medical device connector; and
in the event of receipt of said request failure then logging said failure (470);
said method further comprising:
transmitting (500) a request to said medical device connector (110) for data relating to said medical device (100), said request including said device-Id and authentication key;
in response to said request for data relating to said medical device (100), receiving and displaying one of either data collected (525) from said medical device (100) by said medical device connector (110) in the event said wireless mobile communication device (130) has been authenticated by said medical device connector or an authentication error (535) in the event said wireless mobile communication device is not authenticated by said medical device connector;
transmitting a control action (600) to said medical device connector (110) for controlling said at least one medical device (100), said control action including device-ID and authentication key; and
receiving and displaying one of either data collected (625) from said medical device (100) by said medical device connector (110) in the event said wireless mobile communication device (130) has been authenticated by said medical device connector, and said control action has been successfully implemented, or an authentication error (635) in the event said wireless mobile communication device is not authenticated by said medical device connector,
**characterized by**
said add request including a role,
said role including permission to take said control action,
said confirmation including a medical device connector ID and a role assigned to said wireless mobile communication device,
in the event of receipt of said confirmation then storing (497) said medical device connector ID, assigned role and authentication key in a list of authenticated medical device connectors,
receiving and displaying an authentication error (635) in the event said role does not include permission to take said control action.

7. The method of claim 6 wherein said at least one medical device (100) is an insulin pump, said data collected from said medical device Includes blood glucose level and said control action includes administering insulin via said insulin pump.

8. A wireless mobile communication device (130) to monitor and control at least one medical device (100) via a medical device connector (110), comprising:
a memory (214);
a communication subsystem (200);
a microprocessor (210) executing a software application for causing the wireless mobile communication device (130) to perform the method of claim 6.

9. The wireless mobile communication device (130) of claim 8 wherein said communication subsystem (200) includes one or more receivers, transmitters, antennas, signal processors and other components associated with wireless communications.

10. The method of claim 1 or claim 6, further comprising regulating said transmitting of said control action according to predetermined governance criteria to prevent a race condition between said control action and any other pending control action.

11. The method of claim 10, wherein said governance criteria includes at least one of a threshold for accumulative dose intake, or acknowledgement of completion of said pending control action.

## Patentansprüche

1. Verfahren zum Betreiben eines "medizinische Vorrichtung"-Verbinders (110) zum Überwachen und Steuern zumindest einer medizinischen Vorrichtung (100) unter Verwendung zumindest einer aus einer Vielzahl von drahtlosen mobilen Kommunikationsvorrichtungen (130), das aufweist:
Senden (400) einer Hinzufügen-Anforderung an eine der Vielzahl von drahtlosen mobilen Kommunikationsvorrichtungen (130), wobei die Hinzufügen-Anforderung eine ID und einen Anforderungsschlüssel umfasst;
Empfangen (450) einer Vorrichtungs-ID und eines Authentifizierungsschlüssels von der einen der Vielzahl von drahtlosen mobilen Kommunikationsvorrichtungen (130);
Verarbeiten (455) des Authentisierungsschlüssels, um zu bestimmen, ob die drahtlose mobile Kommunikationsvorrichtung eine authentifizierte drahtlose mobile Kommunikationsvorrichtung (130) ist, und, wenn die drahtlose mobile Kommunikationsvorrichtung nicht authentifiziert ist, dann Senden eines Anforderungs-Fehlschlagens an die drahtlose mobile Kommunikationsvorrichtung;
Ermöglichen zumindest eines aus einem Überwachen von Daten (510) von der zumindest einen medizinischen Vorrichtung (100) und einem Steuern (610) der zumindest einen medizinischen Vorrichtung durch die authentifizierte drahtlose mobile Kommunikationsvorrichtung (130);
wobei das Überwachen von Daten (510) von der zumindest einen medizinischen Vorrichtung (100) weiter aufweist:
Empfangen einer Anforderung (500) von der drahtlosen mobilen Kommunikationsvorrichtung (130) hinsichtlich Daten, die die zumindest eine medizinische Vorrichtung (100) betreffen, wobei die Anforderung die Vorrichtungs-ID und den Authentifizierungsschlüssel umfasst;
in Reaktion auf die empfangene Anforderung hinsichtlich Daten in Bezug auf die medizinische Vorrichtung (100), Prüfen (505) einer Liste von authentifizierten drahtlosen mobilen Kommunikationsvorrichtungen, um zu bestimmen, ob die Vorrichtungs-ID und der Authentifizierungsschlüssel angeben, dass die drahtlose mobile Kommunikationsvorrichtung (130) eine der authentifizierten drahtlosen mobilen Kommunikationsvorrichtungen ist; und
wenn die drahtlose mobile Kommunikationsvorrichtung (130) eine der authentifizierten drahtlosen mobilen Kommunikationsvorrichtungen ist, dann Sammeln (517) der Daten von der zumindest einen medizinischen Vorrichtung und drahtloses Senden (520) der Daten an die drahtlose mobile Kommunikationsvorrichtung (130), und ansonsten Senden eines Authentifizierungsfehlers (530) an die drahtlose mobile Kommunikationsvorrichtung; und
wobei das Steuern (610) der zumindest einen medizinischen Vorrichtung durch die authentifizierte drahtlose mobile Kommunikationsvorrichtung (130) weiter aufweist:
Empfangen einer Steuerungsaktion (600) von der drahtlosen mobilen Kommunikationsvorrichtung (130) zum Steuern der zumindest einen medizinischen Vorrichtung (100), wobei die Steuerungsaktion die Vorrichtungs-ID und den Authentifizierungsschlüssel umfasst; und
in Reaktion auf die empfangene Steuerungsaktion, Prüfen (605, 607) der Liste, um zu bestimmen, ob die Vorrichtungs-ID und der Authentifizierungsschlüssel angeben, dass die drahtlose mobile Kommunikationsvorrichtung (130) eine der authentifizierten drahtlosen mobilen Kommunikationsvorrichtungen ist;
**dadurch gekennzeichnet, dass**
die Hinzufügen-Anforderung eine Rolle bzw. Funktion umfasst,
die Rolle Berechtigungen zum Überwachen und Steuern der zumindest einen medizinischen Vorrichtung umfasst,
die Rolle eine Berechtigung zum Durchführen der Steuerungsaktion umfasst,
das Verarbeiten (455) des Authentifizierungsschlüssels, um zu bestimmen,
ob die drahtlose mobile Kommunikationsvorrichtung eine authentifizierte drahtlose mobile Kommunikationsvorrichtung ist, aufweist:
wenn die drahtlose mobile Kommunikationsvorrichtung authentifiziert ist, dann Zuweisen (480) der Rolle zu der drahtlosen mobilen Kommunikationsvorrichtung, Speichern (485) der Vorrichtungs-ID, des Authentifizierungsschlüssels und der Rolle in der Liste, und Senden (490) einer Bestätigung an die drahtlose mobile Kommunikationsvorrichtung,
das Steuern (610) der zumindest einen medizinischen Vorrichtung durch die authentifizierte drahtlose mobile Kommunikationsvorrichtung (130) weiter aufweist:
wenn die drahtlose mobile Kommunikationsvorrichtung eine der authentifizierten drahtlosen mobilen Kommunikationsvorrichtungen (130) ist und die Rolle eine Berechtigung zum Durchführen der Steuerungsaktion umfasst, dann Senden der Steuerungsaktion (610) an die zumindest eine medizinische Vorrichtung (100), Sammeln von Daten (617) von der zumindest einen medizinischen Vorrichtung nach der Steuerungsaktion, und drahtloses Senden der Daten (620) an die drahtlose mobile Kommunikationsvorrichtung; und ansonsten Senden einer Fehlernachricht (630) an die drahtlose mobile Kommunikationsvorrichtung.

2. Das Verfahren gemäß Anspruch 1, wobei die zumindest eine medizinische Vorrichtung (100) eine Insulinpumpe ist, das Überwachen von Daten ein Überwachen des Blutzuckerspiegels umfasst und das Steuern ein Verabreichen von Insulin über die Insulinpumpe umfasst.

3. Ein "medizinische Vorrichtung"-Verbinder (110) zum Überwachen und Steuern zumindest einer medizinischen Vorrichtung (100) durch zumindest eine aus einer Vielzahl von drahtlosen mobilen Kommunikationsvorrichtungen, der aufweist:
einen Speicher (314);
ein Kommunikationsteilsystem (300);
einen Mikroprozessor (310), der eine Softwareanwendung ausführt, um zu veranlassen, dass der "medizinische Vorrichtung"-Verbinder (110) das Verfahren gemäß Anspruch 1 durchführt; und
einen Datenanschluss (320).

4. Der "medizinische Vorrichtung"-Verbinder gemäß Anspruch 3, wobei das Kommunikationsteilsystem (300) einen oder mehrere Empfänger, Sender, Antennen, Signalprozessoren und andere Komponenten umfasst, die mit drahtlosen Kommunikationen assoziiert sind.

5. Der "medizinische Vorrichtung"-Verbinder gemäß Anspruch 3, wobei der Datenanschluss (320) ein serieller Anschluss ist.

6. Ein Verfahren zum Betreiben einer drahtlosen mobilen Kommunikationsvorrichtung (130) zum Überwachen und Steuern zumindest einer medizinischen Vorrichtung (100), die mit einem "medizinische Vorrichtung"-Verbinder (110) verbunden ist, das aufweist:
Empfangen (405) einer Hinzufügen-Anforderung von dem "medizinische Vorrichtung"-Verbinder (110), wobei die Hinzufügen-Anforderung eine ID und einen Anforderungsschlüssel umfasst;
Verarbeiten (440) der Hinzufügen-Anforderung und in Reaktion Erzeugen einer Vorrichtungs-ID und eines Authentifizierungsschlüssels;
Senden (445) der Vorrichtungs-ID und des Authentifizierungsschlüssels an den "medizinische Vorrichtung"-Verbinder (110);
Empfangen eines aus entweder einer Bestätigung (495), dass die drahtlose mobile Kommunikationsvorrichtung (130) durch den "medizinische Vorrichtung"-Verbinder (110) authentifiziert wurde, oder eines Anforderungs-Fehlschlagens (465) in dem Fall, dass die drahtlose mobile Kommunikationsvorrichtung durch den "medizinische Vorrichtung"-Verbinder nicht authentifiziert wird; und
in dem Fall eines Empfangs des Anforderungs-Fehlschlagens, Protokollieren des Fehlschlagens (470);
wobei das Verfahren weiter aufweist:
Senden (500) einer Anforderung an den "medizinische Vorrichtung"-Verbinder (110) hinsichtlich Daten in Bezug auf die medizinische Vorrichtung (100), wobei die Anforderung die Vorrichtungs-ID und den Authentifizierungsschlüssel umfasst;
in Reaktion auf die Anforderung hinsichtlich Daten in Bezug auf die medizinische Vorrichtung (100), Empfangen und Anzeigen entweder von Daten, gesammelt (525) von der medizinischen Vorrichtung (100) durch den "medizinische Vorrichtung"-Verbinder (110), in dem Fall, dass die drahtlose mobile Kommunikationsvorrichtung (130) durch den "medizinische Vorrichtung"-Verbinder authentifiziert wurde, oder eines Authentifizierungsfehlers (535) in dem Fall, dass die drahtlose mobile Kommunikationsvorrichtung durch den "medizinische Vorrichtung"-Verbinder nicht authentifiziert wurde; Senden einer Steuerungsaktion (600) an den "medizinische Vorrichtung"-Verbinder (110) zum Steuern der zumindest einen medizinischen Vorrichtung (100), wobei die Steuerungsaktion die Vorrichtungs-ID und den Authentifizierungsschlüssel umfasst; und
Empfangen und Anzeigen entweder von Daten, gesammelt (625) von der medizinischen Vorrichtung (100) durch den "medizinische Vorrichtung"-Verbinder (110), in dem Fall, dass die drahtlose mobile Kommunikationsvorrichtung (130) durch den "medizinische Vorrichtung"-Verbinder authentifiziert wurde, und die Steuerungsaktion erfolgreich implementiert wurde, oder eines Authentifizierungsfehlers (635) in dem Fall, dass die drahtlose mobile Kommunikationsvorrichtung durch den "medizinische Vorrichtung"-Verbinder nicht authentifiziert wird,
**dadurch gekennzeichnet, dass**
die Hinzufügen-Anforderung eine Rolle umfasst,
die Rolle eine Berechtigung zum Durchführen der Steuerungsaktion umfasst,
die Bestätigung eine ID des "medizinische Vorrichtung"-Verbinders und eine Rolle umfasst, die der drahtlosen mobilen Kommunikationsvorrichtung zugewiesen wurde,
in dem Fall des Empfangs der Bestätigung, Speichern (497) der ID des "medizinische Vorrichtung"-Verbinders, der zugewiesenen Rolle und des Authentifizierungsschlüssels in einer Liste von authentifizierten "medizinische Vorrichtung"-Verbindern,
Empfangen und Anzeigen eines Authentifizierungsfehlers (635) in dem Fall, dass die Rolle keine Berechtigung zum Durchführen der Steuerungsaktion umfasst.

7. Das Verfahren gemäß Anspruch 6, wobei die zumindest eine medizinische Vorrichtung (100) eine Insulinpumpe ist, die von der medizinischen Vorrichtung gesammelten Daten den Blutzuckerspiegel umfassen und die Steuerungsaktion ein Verabreichen von Insulin über die Insulinpumpe umfasst.

8. Eine drahtlose mobile Kommunikationsvorrichtung (130) zum Überwachen und Steuern zumindest einer medizinischen Vorrichtung (100) über einen "medizinische Vorrichtung"-Verbinder (110), die aufweist:
einen Speicher (214);
ein Kommunikationsteilsystem (200);
einen Mikroprozessor (210), der eine Softwareanwendung ausführt, um zu veranlassen, dass die drahtlose mobile Kommunikationsvorrichtung (130) das Verfahren gemäß Anspruch 6 durchführt.

9. Die drahtlose mobile Kommunikationsvorrichtung (130) gemäß Anspruch 8, wobei das Kommunikationsteilsystem (200) einen oder mehrere Empfänger, Sender, Antennen, Signalprozessoren und andere Komponenten umfasst, die mit drahtlosen Kommunikationen assoziiert sind.

10. Das Verfahren gemäß Anspruch 1 oder Anspruch 6, das weiter ein Regeln des Sendens der Steuerungsaktion gemäß vorgegebenen Lenkungskriterien aufweist, um eine Gleichzeitigkeitsbedingung zwischen der Steuerungsaktion und einer anderen anstehenden Steuerungsaktion zu verhindern.

11. Das Verfahren gemäß Anspruch 10, wobei die Lenkungskriterien zumindest eines aus einer Schwelle für eine Einnahme einer akkumulativen Dosis oder einer Bestätigung eines Abschlusses der anstehenden Steuerungsaktion umfassen.

## Revendications

1. Procédé de fonctionnement d'un connecteur de dispositif médical (110) afin de surveiller et commander au moins un dispositif médical (100) à l'aide d'au moins un dispositif parmi une pluralité de dispositifs de communication mobiles sans fil (130), comprenant les étapes consistant à :
émettre (400) une demande d'addition vers un dispositif parmi ladite pluralité de dispositifs de communication mobiles sans fil (130), ladite demande d'addition incluant un identificateur et une clef de demande ;
recevoir (450) un identificateur de dispositif et une clef d'authentification en provenance dudit un dispositif parmi ladite pluralité de dispositifs de communication mobiles sans fil (130) ;
traiter (455) ladite clef d'authentification afin de déterminer si ledit dispositif de communication mobile sans fil est un dispositif de communication mobile sans fil (130) authentifié et, si ledit dispositif de communication mobile sans fil n'est pas authentifié, émettre un échec de demande vers ledit dispositif mobile de communication sans fil ;
valider au moins soit l'étape consistant à surveiller des données (510) à partir dudit au moins un dispositif médical (100) soit l'étape consistant à commander (610) ledit dispositif médical par ledit dispositif de communication mobile sans fil (130) authentifié ;
dans lequel l'étape de surveillance des données (510) à partir dudit au moins un dispositif médical (100) comprend les étapes consistant à :
recevoir une demande (500) dudit dispositif de communication mobile sans fil (130) pour des données intéressant ledit au moins un dispositif médical (100), ladite demande incluant ledit identificateur de dispositif et ladite clef d'authentification ;
en réponse à la réception de ladite demande de données intéressant ledit dispositif médical (100), examiner (505) une liste de dispositifs de communication mobile sans fil authentifiés afin de déterminer si ledit identificateur de dispositif et ladite clef d'authentification indiquent que ledit dispositif de communication mobile sans fil (130) est l'un des dispositifs de communication mobile sans fil authentifiés ; et
si ledit dispositif de communication mobile sans fil (130) est l'un desdits dispositifs de communication mobile sans fil authentifiés, recueillir (517) lesdites données provenant dudit au moins dispositif médical et émettre par sans fil (520) lesdites données vers ledit dispositif de communication mobile sans fil (130) et sinon émettre une erreur d'authentification (530) vers ledit dispositif de communication mobile sans fil ; et
dans lequel ladite étape de commande (610) dudit au moins dispositif médical par ledit dispositif de communication mobile sans fil (130) authentifié comprend en outre les étapes consistant à :
recevoir une action de commande (600) dudit dispositif de communication mobile sans fil (130) afin de commander ledit au moins un dispositif médical (100), ladite action de commande comprenant l'identificateur de dispositif et la clef d'authentification ; et
en réponse à la réception de l'action de commande, examiner (605, 607) ladite liste afin de déterminer si ledit identificateur de dispositif et ladite clef d'authentification indiquent que ledit dispositif de communication mobile sans fil (130) est l'un desdits dispositifs de communication mobile sans fil authentifiés ;
**caractérisé en ce que** :
ladite demande d'addition inclut un rôle ;
ledit rôle inclut des permissions de surveiller et de commander ledit au moins un dispositif médical ;
ledit rôle inclut une permission d'entreprendre ladite action de commande ;
ladite étape de traitement (455) de ladite clef d'authentification afin de déterminer si ledit dispositif de communication mobile sans fil est un dispositif de communication mobile sans fil authentifié comprend les étapes consistant à :
si ledit dispositif de communication mobile sans fil est authentifié, attribuer (480) le rôle audit dispositif de communication mobile sans fil, enregistrer (485) ledit identificateur de dispositif, ladite clef d'authentification et ledit rôle dans ladite liste et émettre (490) une confirmation vers ledit dispositif de communication mobile sans fil ;
ladite étape de commande (610) dudit au moins un dispositif médical par ledit dispositif de communication mobile sans fil (130) authentifié comprend en outre les étapes consistant à :
si ledit dispositif de communication mobile sans fil est l'un desdits dispositifs de communication mobile sans fil (130) authentifiés et si ledit rôle inclut la permission d'entreprendre ladite action de commande, émettre ladite action de commande (610) vers ledit au moins un dispositif médical (100), recueillir des données (617) provenant dudit au moins un dispositif médical après ladite action de commande et émettre par sans fil lesdites données (620) vers ledit dispositif de communication mobile sans fil et sinon émettre un message d'erreur (630) vers ledit dispositif de communication mobile sans fil.

2. Procédé selon la revendication 1, dans lequel ledit au moins un dispositif médical (100) est une pompe à insuline, ladite surveillance de données inclut la surveillance du niveau de glucose sanguin et ladite commande inclut l'administration d'insuline par ladite pompe à insuline.

3. Connecteur de dispositif médical (110) destiné à surveiller et commander au moins un dispositif médical (100) par au moins un dispositif parmi une pluralité de dispositifs de communication mobile sans fil, comprenant :
une mémoire (314) ;
un sous-système de communication (300) ;
un microprocesseur (310) exécutant une application logicielle afin de commander au connecteur de dispositif médical (110) d'exécuter le procédé selon la revendication 1 ; et
un port de données (320).

4. Connecteur de dispositif médical selon la revendication 3, dans lequel ledit sous-système de communication (300) inclut un ou plusieurs récepteurs, émetteurs, antennes, processeurs de signaux et autres composants associés aux communications sans fil.

5. Connecteur de dispositif médical selon la revendication 3, dans lequel ledit port de données (320) est un port série.

6. Procédé de fonctionnement d'un dispositif de communication mobile sans fil (130) pour surveiller et commander au moins un dispositif médical (100) connecté à un connecteur de dispositif médical (110), comprenant les étapes consistant à :
recevoir (405) une demande d'addition dudit connecteur de dispositif médical (110), ladite demande d'addition incluant un identificateur et une clef de demande ;
traiter (440) ladite demande d'addition et en réponse produire un identificateur de dispositif et une clef d'authentification ;
émettre (445) ledit identificateur de dispositif et ladite clef d'authentification vers ledit connecteur de dispositif médical (110) ;
recevoir soit une confirmation (495) que ledit dispositif de communication mobile sans fil (130) a été suffisamment authentifié par ledit connecteur de dispositif médical (110), soit un échec de la demande (465) si ledit dispositif de communication mobile sans fil n'est pas authentifié par ledit connecteur de dispositif médical ; et
dans le cas de la réception dudit échec de la demande, enregistrer ledit échec (470) dans un journal ;
ledit procédé comprenant en outre les étapes consistant à :
émettre (500) vers ledit connecteur de dispositif médical (110) une demande portant sur des données intéressant ledit dispositif médical (100), ladite demande incluant ledit identificateur de dispositif et ladite clef d'authentification ;
en réponse à ladite demande de données intéressant ledit dispositif médical (100), recevoir et afficher soit des données recueillies (525) provenant dudit dispositif médical (100) par ledit connecteur de dispositif médical (110) si ledit dispositif de communication mobile sans fil (130) a été authentifié par ledit connecteur de dispositif médical, soit une erreur d'authentification (535) dans le cas où ledit dispositif de communication mobile sans fil n'a pas été authentifié par ledit connecteur de dispositif médical ;
émettre une action de commande (600) vers ledit connecteur de dispositif médical (110) afin de commander ledit au moins dispositif médical (100), ladite action de commande comprenant l'identificateur de dispositif et la clef d'authentification ; et
recevoir et afficher soit les données recueillies (625) provenant dudit dispositif médical (100) par ledit connecteur de dispositif médical (110) si ledit dispositif de communication mobile sans fil (130) a été authentifié par ledit connecteur de dispositif médical et si ladite action de commande a été mise en oeuvre avec succès, soit une erreur d'authentification (635) dans le cas où ledit dispositif de communication mobile sans fil n'a pas été authentifié par ledit connecteur de dispositif médical ;
**caractérisé en ce que** :
ladite demande d'addition inclut un rôle ;
ledit rôle inclut une permission d'entreprendre ladite action de commande ;
ladite confirmation inclut un identificateur de connecteur de dispositif médical et un rôle attribué audit dispositif de communication mobile sans fil ;
dans le cas où ladite confirmation a été reçue, enregistrer (497) ledit identificateur de connecteur de dispositif médical, ledit rôle attribué et ladite clef d'authentification dans une liste de connecteurs de dispositif médical authentifiés ;
recevoir et afficher une erreur d'authentification (635) dans le cas où ledit rôle n'inclut pas la permission d'entreprendre ladite action de commande.

7. Procédé selon la revendication 6, dans lequel ledit au moins un dispositif médical (100) est une pompe à insuline, lesdites données recueillies dudit dispositif médical incluent le niveau de glucose sanguin et ladite commande inclut l'administration d'insuline par ladite pompe à insuline.

8. Dispositif de communication mobile sans fil (130) destiné à surveiller et à commander au moins un dispositif médical (100) via un connecteur de dispositif médical (110), comprenant :
une mémoire (214) ;
un sous-système de commutation (200) ;
un microprocesseur (210) exécutant une application logicielle afin de commander au dispositif de communication mobile sans fil (130) d'exécuter le procédé selon la revendication 6.

9. Dispositif de communication mobile sans fil (130) selon la revendication 8, dans lequel ledit sous-système de commutation (200) comprend un ou plusieurs récepteurs, émetteurs, antennes, processeurs de signaux et autres composants associés aux communications sans fil.

10. Procédé selon la revendication 1 ou la revendication 6, comprenant en outre l'étape consistant à réguler ladite émission de ladite action de commande en fonction d'un critère de gouvernance prédéterminé afin d'empêcher une situation de compétition entre ladite action de commande et toute autre action de commande en cours.

11. Procédé selon la revendication 10, dans lequel ledit critère de gouvernance comprend au moins soit un seuil de dose administrée cumulée, soit un accusé de réception indiquant l'achèvement de ladite action de commande en cours.
